# EUROPEAN PATENT SPECIFICATION

(11) **EP 2 319 549 B1**
(45) Date of publication and mention of the grant of the patent: **15.05.2013**
(21) Application number: 10184118.7
(22) Date of filing: 30.09.2010
(51) Int. Cl.: A61M 1/00, A61M 27/00

(54) **Chemically coated screen for use with hydrophobic filters**
Chemisch beschichtetes Sieb zur Verwendung mit hydrophoben Filtern
Écran chimique revêtu pour une utilisation avec des filtres hydrophobes

(30) Priority: 05.11.2009 US 258253 P
(43) Date of publication of application: 11.05.2011
(73) Proprietor: Covidien LP, Mansfield, MA 02048 (US)
(72) Inventor: Vess, Mark A., Hanson, Massachusetts 02341 (US); Aggarwal, Dinesh, Franklin, Massachusetts 02038 (US)
(74) Representative: Gray, James

(56) References cited:
- JP-A- 2006 084 129
- US-A1- 2004 050 254
- US-A1- 2005 119 737
- US-A1- 2009 221 990

## Description

### BACKGROUND

### 1. Technical Field

The present disclosure relates to treating an open wound, and, more specifically, relates to a wound therapy system including a chemically treated screen for releasing compounds for the long term maintenance of hydrophobic filters used in negative pressure wound therapy pumps.

### 2. Description of Related Art

Wound closure involves the migration of epithelial and subcutaneous tissue adjacent the wound towards the center and away from the base of the wound until the wound closes. Unfortunately, closure is difficult with large wounds, chronic wounds or wounds that have become infected. In such wounds, a zone of stasis (i.e. an area in which localized swelling of tissue restricts the flow of blood to the tissues) forms near the surface of the wound. Without sufficient blood flow, the epithelial and subcutaneous tissues surrounding the wound not only receive diminished oxygen and nutrients, but, are also less able to successfully fight microbial infection and, thus, are less able to close the wound naturally. Such wounds have presented difficulties to medical personnel for many years.

Negative pressure wound therapy, also known as suction or vacuum therapy, has been used in treating and healing wounds. Application of negative pressure, e.g. reduced or subatmospheric pressure, to a localized reservoir over a wound has been found to assist in closing the wound by promoting blood flow to the area, stimulating the formation of granulation tissue, and encouraging the migration of healthy tissue over the wound. Negative pressure may also inhibit bacterial growth by drawing fluids from the wound such as exudates, which may tend to harbor bacteria. This technique has proven particularly effective for chronic or healing-resistant wounds, and is also used for other purposes such as post-operative wound care.

Generally, negative pressure therapy provides for a wound to be covered to facilitate suction at the wound area. A conduit is introduced through the wound covering to provide fluid communication to an external vacuum source. Atmospheric gas, wound exudates, or other fluids may thus be drawn from the reservoir through the fluid conduit to stimulate healing of the wound. Exudates drawn from the reservoir may be deposited in a collection canister. The canister of the wound therapy system may require disconnection or replacement for a variety of reasons, such as when filled with exudates, or if exudates escape and/or clog the filter or electronics of the system. It would be advantageous to provide a collection canister which seals the contents therein and precludes the escape of the contents when the canister is tilted or oriented with the filter side down while inhibiting clogging of the filter with exudates in the event that exudates contact the filter.

US2009/0221990 discloses a reduced pressure treatment system including a porous pad positioned at a tissue site and a canister having a collection chamber, an inlet and an outlet. US2004/0050254 is directed to an air purifying filter which includes a modified enzyme immobilized on a surface of a carrier. US2005/0119737 is directed to an ocular implant device that is insertable into either the anterior or posterior chamber of the eye to drain aqueous humor and/or to introduce medications. JP2006084129 is directed to an air cleaner for a building having a filter with at least one of an enzyme and a protein modifying agent for inactivating allergens.

### SUMMARY

According to the present invention, there is provided a system to promote the healing of an exuding wound, which comprises:
a wound dressing dimensioned for positioning relative to a wound bed (10) of a subject; and
a subatmospheric pressure mechanism including:
   a housing;
   a vacuum source disposed in the housing and associated with a vacuum port;
   a collection canister defining an internal chamber in fluid communication with the vacuum source through the vacuum port and with the wound dressing for collecting exudates removed from the wound bed under influence of the vacuum source;
   a hydrophobic filter in fluid communication with the vacuum source and the internal chamber of the collection canister, the filter adapted to prevent exudates from reaching the vacuum source while allowing passage of air to the vacuum source; characterised in that the system further comprises:
   a screen including compounds for cleaving proteins in the exudates, the screen disposed proximally of the filter to prevent clogging of the filter by the exudates in the collection canister.

The compounds immobilized on the screen include proteases and nucleases for breaking down proteins. The compounds may be coated on the screen by powder or crystalline coating or may be molded onto the surface of the screen. Upon contact with exudates, the compounds may activate and degrade proteins and other compounds contained therein.

### BRIEF DESCRIPTION OF THE DRAWINGS

Various embodiments of the wound dressing system of the present disclosure are described herein with reference to the drawings wherein:

**FIG. 1** is a view in partial cross-section of a wound therapy system of the present disclosure illustrating the wound dressing and the subatmospheric pressure mechanism;

**FIGS. 2A** and **2B** are cross-sectional views of alternate embodiments of the subatmospheric pressure mechanism of the wound therapy system of the present disclosure;

**FIG. 3** is a perspective view of a canister insert in accordance with the principles of the present disclosure;

**FIG. 4** is a schematic illustration of a filter and screen combination for use with the wound therapy system of the present disclosure; and

**FIG. 5** is a schematic illustration of an alternate filter and screen combination for use with the wound therapy system of the present disclosure.

### DESCRIPTION OF THE EMBODIMENTS

The wound therapy system of the present disclosure promotes healing of a wound via the use of a wound dressing and a subatmospheric pressure mechanism. Generally, the subatmospheric pressure mechanism applies subatmospheric pressure to the wound to effectively remove wound fluids or exudates captured within the boundary of the composite wound dressing, and to increase blood flow to the wound bed and enhance cellular stimulation of epithelial and subcutaneous tissue. The wound therapy system may be entirely portable, i.e., it may be worn or carried by the subject such that the subject may be completely ambulatory during the therapy period. The wound therapy system including the subatmospheric pressure mechanism and components thereof may be entirely reusable or may be entirely disposable after a predetermined period of use or may be individually disposable whereby some of the components are reused for a subsequent therapy application.

The wound therapy system of the present disclosure promotes healing of a wound in conjunction with subatmospheric negative pressure therapy. The system may incorporate a variety of wound dressings, subatmospheric pressure sources and pumps and collection canisters. The attached figures illustrate exemplary embodiments of the present disclosure and are referenced to describe the embodiments depicted therein. Hereinafter, the disclosure will be described by explaining the figures wherein like reference numerals represent like parts throughout the several views.

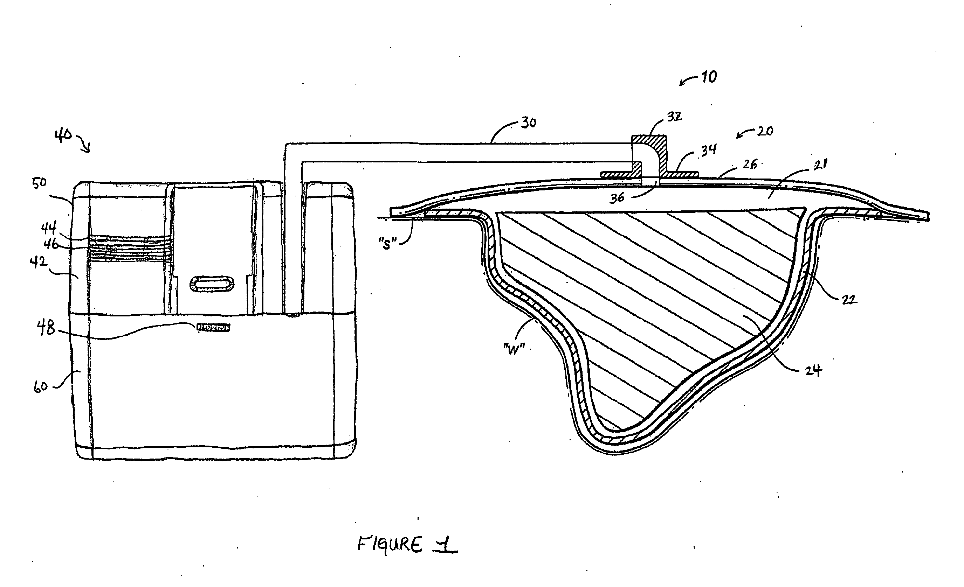

Referring initially to **FIG. 1****,** the wound therapy system **10** according to the present disclosure is illustrated for use on a wound "w" surrounded by healthy skin "s." Wound therapy system **10** includes composite wound dressing **20** and subatmospheric pressure mechanism **40** in fluid communication with the wound dressing **20** through conduit **30.**

Wound dressing **20** is positioned relative to the wound "w" to define a reservoir **21** in which a negative pressure appropriate to stimulate healing may be maintained. Wound dressing **20** may include several components, namely, wound contact layer or member **22,** a wound packing member or filler **24** supported by the contact member **22,** and outer layer or cover member **26.** Wound contact member **22** is adapted to substantially conform to the topography of a wound bed "w." Wound contact member **22** may be substantially porous or perforated to permit exudates to pass from the wound bed "w" through the wound contact member **22.** The passage of wound exudates through the wound contact member **22** may be unidirectional such that wound exudates do not flow back to the wound bed "w." Unidirectional flow may be encouraged by directional apertures formed in contact member **22,** lamination of materials having absorption properties differing from those of contact member **22,** or by selection of materials that promote directional flow. A non-adherent material may be selected such that contact member **22** does not tend to cling to wound bed "w" or surrounding material when it is removed. Exemplary materials that may be used as a contact member **22** are sold under the trademarks XEROFORM^{®} and CURITY^{®}, offered by Tyco Healthcare Group LP (d/b/a Covidien).

Wound packing member **24** of wound dressing **20** is intended to transfer wound fluid and exudates. Wound packing member **24** is conformable to assume the shape of any wound bed "w" and may be packed up to the level of healthy skin "s." Wound packing member **24** may be pre-formed in any shape and size or may be custom fit by cutting the packing member **24** to a desired shape and/or size. Wound packing member **24** may be treated with agents to promote healing of the wound, such as polyhexamethylene biguanide to decrease the incidence of infection, and other substances having clinical use, such as a dye. Suitable materials for wound packing member **24** are sold under the trademarks KERLIX^{®}, EXCILON^{®} and WEBRIL^{®}, all by Tyco Healthcare Group LP (d/b/a Covidien).

Outer member or wound covering **26** encompasses the perimeter of the wound dressing **20** to surround wound bed "w" and to provide a liquid and/or fluid tight seal around the perimeter "p" of the wound bed "w." For instance, the sealing mechanism may be any adhesive bonded to the perimeter of wound covering **26.** One exemplary material that may be used as an adhesive dressing is sold under the trademark CURAFOAM^{®} by Tyco Healthcare Group LP (d/b/a Covidien). Thus, wound covering **26** may act as both a microbial barrier and a fluid barrier to prevent contaminants from entering wound bed "w" and for maintaining the integrity thereof.

Wound covering **26** is typically a flexible material, e.g., resilient or elastomeric, that seals the top of wound dressing **20** to prevent passage of liquids, fluids, or contamination to and from the wound dressing **20.** Wound covering **26** may be formed from a moisture vapor permeable membrane to promote the exchange of oxygen moisture between the wound bed "w" and atmosphere. A membrane that provides a sufficient moisture vapor transmission rate is a transparent membrane sold under the trademark POLYSKIN^{®} II by Tyco Healthcare Group LP (d/b/a Covidien). A transparent membrane permits an assessment of wound conditions without requiring removal of the wound covering **26.** Alternatively, wound covering **26** may comprise an impermeable membrane or a substantially rigid membrane.

Wound covering **26** may include a port or connector **32** in fluid communication with the wound dressing **20** to facilitate connection of wound dressing **20** to conduit or tubing **30.** Conduit **30** defines a fluid flow path leading through wound therapy system **10.** Connector **32** may be configured as a rigid or flexible, low-profile component, and may be adapted to receive conduit **30** in a releasable and fluid tight manner. An adhesive on the underside of flange **34** of connector **32** may provide a mechanism for affixing the conduit **30** to the dressing or alternatively, flange **34** may be positioned within reservoir **21** such that an adhesive on an upper side of the flange **34** affixes the conduit **30.** However it is affixed to wound dressing **20,** a hollow interior **36** of connector **32** provides fluid communication between conduit **30** and the interior of wound dressing **20,** such as reservoir **21.**

Connector **32** may have a valve (not shown) built therein or in line with conduit **30,** e.g., a one-way valve to permit exudates to flow in one direction only, i.e., away from wound dressing **20** toward subatmospheric pressure mechanism **40.** Connector **32** may be provided as a pre-affixed component of wound dressing **20,** as a component of conduit **30** or entirely separate and connected thereto by conventional means. Alternatively, connector **32** may be eliminated if other provisions are made for providing fluid communication between wound dressing **20** and conduit **30.**

Conduit **30** extends from wound dressing **20** to subatmospheric pressure mechanism **40.** Any suitable conduit may be used including those fabricated from flexible elastomeric or polymeric materials. Conduit **30** may connect to subatmospheric pressure mechanism **40** or other system components by conventional air tight means such as friction fit, bayonet coupling, or barbed connectors. The conduit connections may be made permanent, or alternatively a quick-disconnect or other releasable means may be used to provide some adjustment flexibility to the apparatus.

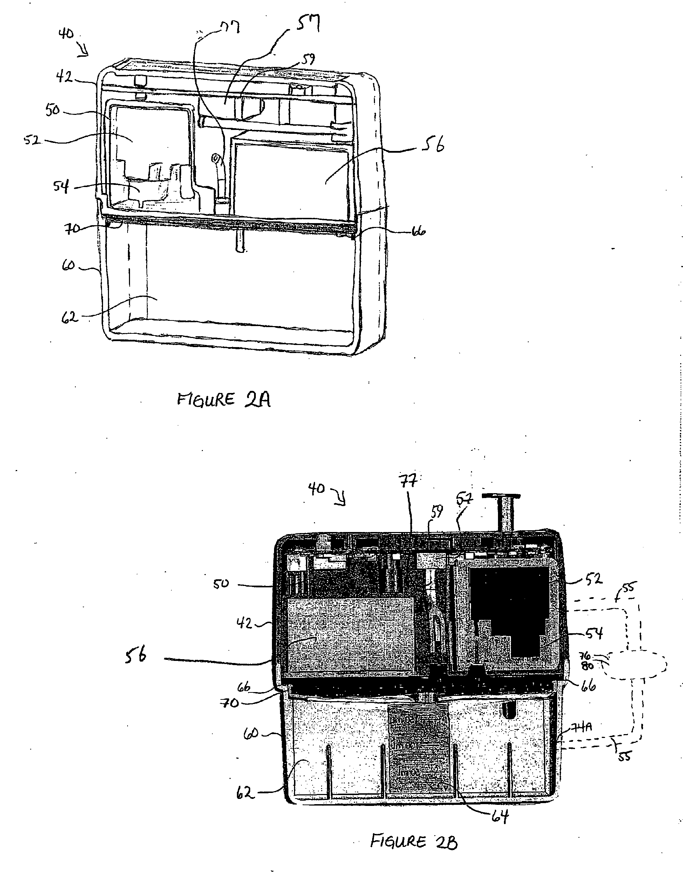

Referring now to **FIGS. 2A** and **2B****,** in conjunction with **FIG. 1****,** subatmospheric pressure mechanism 40 will be discussed. Subatmospheric pressure mechanism **40** includes housing **42,** control unit **50** disposed within the housing **42,** and collection canister **60.** Housing **42** may be any suitable rigid member adapted for donning by the subject. Control unit **50** may incorporate vacuum source or pump **52,** actuator or motor **54** for activating the vacuum source **52,** and power source 56. Vacuum source or pump **52** generates or otherwise provides negative pressure to wound therapy system **10.** Vacuum source or pump **52** may be a pump of the diaphragmatic, peristaltic or bellows type or the like, in which the moving part(s) draw exudates out of the wound bed "w" into the wound dressing **20** by creating areas or zones of decreased pressure e.g., vacuum zones with the wound dressing **20** appropriate to stimulate healing of the wound. This area of decreased pressure may communicate with the wound bed "w" to facilitate removal of the fluids therefrom and into packing member **24.**

Vacuum source or pump **52** may be a miniature pump or micropump that may be biocompatible and adapted to maintain or draw adequate and therapeutic vacuum levels. The vacuum level of subatmospheric pressure achieved may be in the range of about 20 mmHg to about 500 mmHg. In embodiments, the vacuum level may be about 75 mmHg and about 125 mmHg, or between about 30 mmHg and 80 mmHg. Vacuum source or pump **52** is actuated by actuator **54** which may be any means known by those skilled in the art, including, for example, AC motors, DC motors, voice coil actuators, solenoids, etc. In embodiments, actuator **54** may be incorporated within pump **52.**

Power source **56** may be disposed within housing **42** or separately mountable to the housing **42.** A suitable power source **56** includes alkaline batteries, wet cell batteries, dry cell batteries, nickel cadmium batteries, solar generated means, lithium batteries, NiMH batteries (nickel metal hydride) each of which may be of the disposable or rechargeable variety.

Subatmospheric pressure mechanism **40** may also include a pressure transducer 57 which may be attached to a printed circuit board (PCB) **59.** Within the PCB **59** is software or circuitry that powers the pressure transducer **57** and receives its pressure signals (i.e., electrical signals indicative of the negative pressure being measured).

Housing **42** may further include vent portal **44** configured to vent exhaust air from vacuum source or pump **52** through an exhaust port (not shown). Vent portal **44** extends from housing **42** and may be directly connected to vacuum source **52.** It is also envisioned that vent portal **44** may exhaust air from within housing **42** rather than directly from vacuum source **52.** Vent portal **44** may include filter **46** extending across the vent portal **44.** Filter **46** may be a bacterial filter including charcoal or other odor absorbing materials to help prevent emission of bacteria from housing **42.**

Collection canister **60** collects exudates removed from the wound bed "w' during therapy through conduit or tubing **30.** Collection canister **60** is associated with housing **42** and may be incorporated within the housing **42** or releasably connected to the housing **42** by conventional means. Housing **42** and collection canister **60** of subatmospheric pressure mechanism **40** may be releasably coupled via mating members **48.** Mechanisms for selective coupling and decoupling of housing **42** and collection canister **60** include fasteners, latches, clips, straps, bayonet mounts, magnetic couplings, and other devices for selective mating of housing **42** and collection canister **60.**

Collection canister **60** may comprise any container suitable for containing wound fluids and is substantially rigid defining an internal chamber **62** in fluid communication with tubing **30.** In the alternative, collection canister **60** may be relatively flexible. Collection canister **60** may contain an absorbent material to consolidate or contain the wound drainage or debris, such as silica gel. In embodiments, at least a portion of collection canister **60** may be transparent to assist in evaluating the color, quality, or quantity of wound exudates. A transparent portion or window **64** may thus assist in determining the remaining capacity of the canister **60** or when the canister **60** should be replaced.

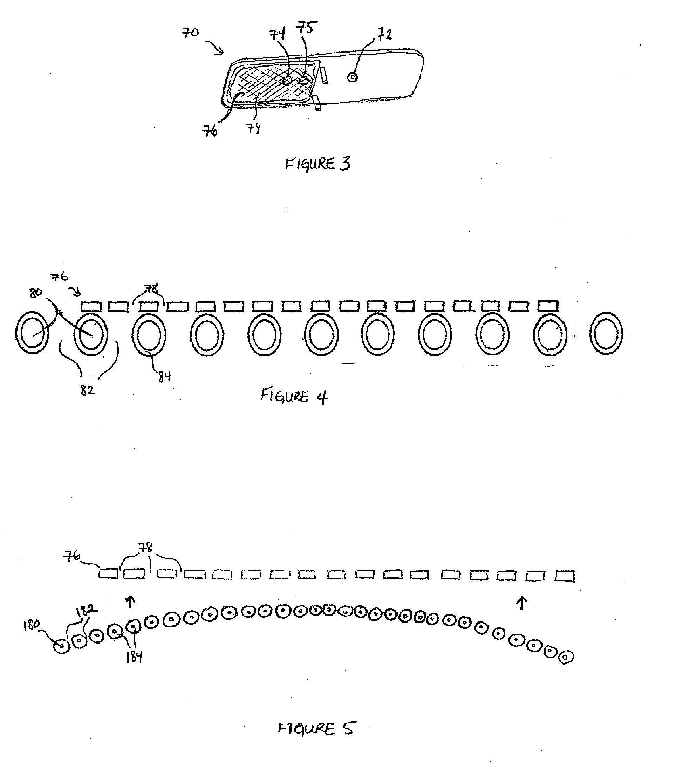

Collection canister **60** may include a canister insert **70.** Referring now to **FIG. 3****,** in conjunction with **FIGS. 2A** and **2B****,** canister insert **70** is dimensioned to fill the opening of canister **60** and be placed within internal chamber **62** of canister **60** until it engages a lip **66** around at least a portion of the peripheral inner edge of canister **60** or frictionally engages the inner walls of the canister in a fluid tight, yet releasable manner. Canister insert **70** include fluid inlet **72,** suction port **74** (shown in phantom), and a pressure transducer port **75** (shown in phantom). The suction port **74** and the pressure transducer port **75** are disposed beneath filter **76.** Fluid inlet **72** depends from a planar segment of canister insert **70** and is configured to operably engage conduit **30.** Fluid inlet **72** may be connectable with conduit **30** by conventional air and fluid tight means, such as those described above, and terminates within internal chamber **62** to deposit exudates conveyed by the conduit **30** into the internal chamber **62.** In embodiments, fluid inlet **72** may contain a luer lock or other connector within the purview of those skilled in the art to secure the end of conduit **30** with the fluid inlet **72.** It is envisioned that fluid inlet **72** is configured to receive a cap in order to prevent leakage of exudates and odor from internal chamber **62** of collection canister **60** when housing **42** is separated from the canister **60.**

Suction port **74** is in fluid communication with vacuum source or pump **52** and may be an opening defined in canister insert **70.** Pump **52** creates a vacuum within internal chamber **62** of collection canister **60** by drawing air through suction port **74.** Pressure transducer port **75** is in fluid communication with pressure transducer **57** through tube **77** and permits the monitoring of pressure levels within internal chamber **62** of collection canister **60.**

Referring now to **FIG. 4****,** canister insert **70** includes a filter **76,** such as a hydrophobic membrane or baffling, including pores **78** to prevent exudates from being aspirated into pump **52.** Filter **76** is attached to canister insert **70** through conventional means, such as mechanical binding. The filter **76** may be dimensioned to span the lower surface of canister insert **70** to cover suction port **76** and pressure transducer port **75.** The filter **76** is disposed adjacent to or within suction port **74** such that suction port **74** passes air between vacuum source **52** and the canister **60** through filter **76** while keeping the contents of the canister from reaching the vacuum pump **52** or other components of control unit **50.** The filter **76** also prevents migration of the fluids or exudates into the pressure transducer port **75** and pressure transducer **57.**

The hydrophobic nature of the filter **76** allows the canister **60** to be oriented in a way other than with the pump **52** above the canister **60,** such as on the side of the canister **60** or tipped, without exudates in the canister **60** being aspirated into the pump **52.** Some portion of the surface of the filter **76** remains uncovered, thereby allowing continued flow of air to vacuum pump **52.**

The pores **78** of the filter **76**, however, may become clogged over time as a result of exudates coming in contact with the filter's surface. Protein strands and other exudates compounds may become lodged in the pores **78** of the filter **76**, thereby reducing air flow through filter **76.** A chemically treated screen **80** may be disposed adjacent to the surface of the filter **76** facing the canister **60** to break down the proteins and other compounds in the exudates, and proteins thereof trapped in the filter's pores **78,** to preserve the ability of the screen **80** to function over time. Cleavage of large compounds in the exudates, such as proteins, nucleic acids, lipids, and polysaccharides, into smaller units may prevent these compounds from becoming trapped in the filter's pores **78.**

The screen **80** may comprise a fine mesh of plastic or other inert material. Exemplary materials include, but are not limited to, polyolefins (such as polyethylene and polypropylene); polyesters (such as polyethylene terephthalate and polybutylene terephthalate); acrylic polymers and copolymers; vinyl halide polymers and copolymers (such as polyvinyl chloride); polyamides (such as nylon 4, nylon 6, nylon 6.6, nylon 610, nylon 11, nylon 12 and polycaprolactam); polyurethanes, silicones, rayon, and spandex.

The screen **80** includes openings **82** that measure about 0.254mm (0.01 inches) to about 0.762mm (0.03 inches) across. In embodiments, the size of the openings **82** are 0.508mm (0.02 inches).

The screen **80** also contains compounds **84** which are immobilized to the surface of the screen **80.** Compounds **84** include various proteases, nucleases, and proteins which have the ability to cleave peptide bonds thus reducing the molecular weight of a protein strand and creating smaller sized peptides. By reducing the size of the proteins in the exudates, clogging of the hydrophobic filter **76** may be eliminated or at least diminished so that the filter **76** remains functional. Examples of proteases include papain, trypsin, cathepsin, plasmin, pepsin, chymotrypsin, thermolysin, carboxypeptidase Y, Glu-C, Asp-N, Lys-C, and combinations thereof, such as Glu-C/Trypsin, Glu-C/Chymotrypsin, and Trypsin/Asp-N, for cleavage at different sites along the protein. A variety of nucleases, including exo- and/or endo- nucleases, may be used with screen **80** as is within the purview of those skilled in the art. Nucleases include a variety of restriction enzymes, DNA, and RNA which can catalyze various reactions, such as the cleavage of DNA, hydrolysis of RNA, and combinations thereof. Moreover, other bioactive agents may be combined with the compounds **84** or coated on the screen **80,** such as anti-adhesives, antimicrobials, anti-infectives, anti-thrombotics, and other substances which may aid in maintaining a clean filter as is within the purview of those skilled in the art.

The compounds **84** are coated, bonded, or otherwise applied to screen **80** by any method within the purview of those skilled in the art. Exemplary methods include, for example, powder coating, crystalline coating, and molding. Powder coating may include dry coatings of compound **84** which do not require a solvent. Crystalline coatings may include a solution of compound **84** and solvent which may be deposited on the screen **80** via dipping, spinning, brushing, spraying, and other means within the purview of those skilled in the art. Through the use of a polymerization, condensation, or heating process, the compounds **84** form a crystalline coating.

The screen **80** may be adapted and configured to conform to the surface of the filter **76** to ensure contact with the filter **76** over its entire surface. The screen **80** may be placed in close proximity to the filter **76,** and in embodiments, may be mechanically bonded to filter **76.** The screen **80** may cover substantially the entire filter **76** or, in embodiments, may cover a majority of the filter **76.**

In embodiments, screen **180** is fabricated with a slight curvature over its surface as illustrated in **FIG. 5****.** This curvature allows the screen **180** to flatten out onto the filter **76** upon movement of exudates in the direction of the arrows to ensure contact over a majority of the filter's surface. This configuration places the compounds **184** of screen **180** in contact with or in close proximity to a large number of pores **78** of filter **76.** As exudates come in contact with the screen **180** and filter **76,** some of the compounds **184** of screen **180** may dissolve, or otherwise become active, and remain in the pores **78** of the filter **76.** Although the compounds **184** may not reach all of the pores **78,** enough of the pores **78** remain open so as to preserve the functionality of the filter **76** over time and exposure to exudates. The compounds **184** may also attach to any protein deposits that may come in contact with and/or adhere to the surface of the screen **180.** As the exudates are sloshed around the canister **60,** the movement of the fluid may allow any accumulated debris to be washed away from the screen **180.** In embodiments, the curvature of the screen **180** may be concave or convex, and likewise, the filter **76** may also possess a slight convex or concave curvature independent of, or complimentary to, the curvature of the screen **180.**

Alternatively, the filter **76** and screen **80** combination may be disposed in another intermediary location between pump **52** and internal chamber **62** of canister **60,** e.g., with the filter **76** and the screen **80** external of internal chamber **62.** As illustrated in phantom in **FIG. 2B****,** filter **76** and screen **80** may be disposed along tube **55** which connects suction port **74A** of canister **60** with vacuum pump **52.**

In an exemplary embodiment of use, the wound dressing **20** is placed adjacent the wound bed "w" and connected to subatmospheric pressure mechanism **40** via tubing **30,** as illustrated in **FIG. 1****.** Housing **42** and canister **60** are connected if not already connected to each other. Control unit **50** of subatmospheric pressure mechanism **40** is then activated creating a reduced pressure state within wound dressing **20.** Vacuum source or pump **52** may be set at a specific set point whereby the pump will begin to draw vacuum until it achieves the set point as detected, e.g., by a pressure transducer. As the pumping progresses, exudates are collected and directed to collection canister **60.** The vacuum reading at the pump **52** will stay at this level until the set point is changed, the pump is turned off, or there is a major leak in the system that overcomes the pump's ability to continue to achieve this level. Subatmospheric pressure therapy may be continuous or intermittent.

In the event that exudates contact filter **76,** such as by tilting or inversion of the canister **60**, the screen **80** provides protection to the filter **76** by dissolving and/or degrading proteins and compounds in the exudates that may accumulate on the surface of the screen **80** and/or filter **76.** The screen **80** effectively forms an active protective barrier over the hydrophobic filter **76.**

While the disclosure has been illustrated and described, it is not intended to be limited to the details shown, since various modifications and substitutions can be made. As such, further modifications and equivalents of the disclosure herein can occur to persons skilled in the art, and all such modifications and equivalents are believed to be within the scope of the disclosure as defined by the following claims.

## Claims

1. A system to promote the healing of an exuding wound, which comprises:
a wound dressing (20) dimensioned for positioning relative to a wound bed (10) of a subject; and
a subatmospheric pressure mechanism (40) including:
a housing (42);
a vacuum source (52) disposed in the housing (42) and associated with a vacuum port (74);
a collection canister (60) defining an internal chamber (62) in fluid communication with the vacuum source (52) through the vacuum port (74) and with the wound dressing (20) for collecting exudates removed from the wound bed (10) under influence of the vacuum source (52);
a hydrophobic filter (76) in fluid communication with the vacuum source (52) and the internal chamber (62) of the collection canister (60), the filter (76) adapted to prevent exudates from reaching the vacuum source (52) while allowing passage of air to the vacuum source (52); **characterised in that** the system further comprises:
a screen (80) including compounds for cleaving proteins in the exudates, the screen (80) disposed proximally of the filter (76) to prevent clogging of the filter (76) by the exudates in the collection canister (60).

2. The system according to claim **1**, wherein the compounds are proteases.

3. The system according to claim **2**, wherein the proteases are one of papain, trypsin, cathepsin, thermolysin, plasmin, pepsin, chymotrypsin, carboxypeptidase Y, Glu-C, Asp-N, Lys-C, and combinations thereof.

4. The system according to claim **1**, wherein the compounds are nucleases.

5. The system according to claim **4**, wherein the nucleases are one of DNA, RNA, and combinations thereof.

6. The system according to claim **4**, wherein the nucleases are exo- and/or endonucleases.

7. The system according to any preceding claim, wherein the screen (80) is mechanically bonded to the filter (76).

8. The system according to any preceding claim, wherein the screen (80) is disposed adjacent to the filter (76).

9. The system according to any preceding claim, wherein the filter (76) and the screen (80) are disposed in a canister inlet (72).

10. The system according to claim **9**, wherein the filter (76) is disposed substantially planar to the canister inlet (72).

11. The system according to claim **10**, wherein the screen (180) is disposed at a slight curvature relative to the filter (76).

12. The system according to any preceding claim, wherein the compounds are immobilized on the screen (80,180) via one of powder coating, crystalline coating, and molding.

## Patentansprüche

1. System zur Förderung der Heilung einer exsudierenden Wunde, umfassend:
einen Wundverband (20), der solche Abmessungen aufweist, dass er relativ zu einem Wundbett (10) einer Person platziert werden kann; und
einen Unterdruckmechanismus (40) mit:
einem Gehäuse (42);
einer im Gehäuse (42) angeordneten und mit einer Vakuumanschlussöffnung (74) verbundenen Vakuumquelle (52);
einem Sammelbehälter (60), der eine Innenkammer (62) in Flüssigkeitsverbindung mit der Vakuumquelle (52) durch die Vakuumanschlussöffnung (74) und mit dem Wundverband (20) zur Sammlung von aus dem Wundbett (10) unter dem Einfluss der Vakuumquelle (52) entfernten Exsudaten definiert;
einen hydrophoben Filter (76) in Flüssigkeitsverbindung mit der Vakuumquelle (52) und der Innenkammer (62) des Sammelbehälters (60), wobei der Filter (76) dazu ausgelegt ist zu verhindern, dass Exsudate die Vakuumquelle (52) erreichen, während er den Durchgang von Luft zur Vakuumquelle (52) gestattet; **dadurch gekennzeichnet, dass** das System ferner Folgendes umfasst:
ein Sieb (80) mit Verbindungen zur Aufspaltung von Proteinen in den Exsudaten, wobei das Sieb (80) proximal vom Filter (76) angeordnet ist, um Verstopfen des Filters (76) durch die Exsudate im Sammelbehälter (60) zu verhindern.

2. System nach Anspruch 1, worin die Verbindungen Proteasen sind.

3. System nach Anspruch 2, worin die Proteasen Papain, Trypsin, Cathepsin, Thermolysin, Plasmin, Pepsin, Chymotrypsin, Carboxypeptidase Y, Glu-C, Asp-N, Lys-C oder Kombinationen davon sind.

4. System nach Anspruch 1, worin die Verbindungen Nukleasen sind.

5. System nach Anspruch 4, worin die Nukleasen DNA, RNA oder Kombinationen davon sind.

6. System nach Anspruch 4, worin die Nukleasen Exo- und/oder Endonukleasen sind.

7. System nach einem der vorhergehenden Ansprüche, worin das Sieb (80) mechanisch mit dem Filter (76) verbunden ist.

8. System nach einem der vorhergehenden Ansprüche, worin das Sieb (80) neben dem Filter (76) angeordnet ist.

9. System nach einem der vorhergehenden Ansprüche, worin der Filter (76) und das Sieb (80) in einem Behältereinlass (72) angeordnet sind.

10. System nach Anspruch 9, worin der Filter (76) im Wesentlichen ebenflächig zum Behältereinlass (72) angeordnet ist.

11. System nach Anspruch 10, worin das Sieb (180) in einer kleinen Krümmung relativ zum Filter (76) angeordnet ist.

12. System nach einem der vorhergehenden Ansprüche, worin die Verbindungen durch Pulverbeschichtung, kristalline Beschichtung oder Formung auf dem Sieb (80, 180) immobilisiert sind.

## Revendications

1. Système pour favoriser la guérison d'une plaie exudative, qui comprend :
un pansement (20) dimensionné pour le positionnement par rapport au lit d'une plaie (10) d'un sujet ; et
un mécanisme à pression sous-atmosphérique (40) comprenant :
un boîtier (42) ;
une source de vide (52) disposée dans le boîtier (42) et associée à un orifice de mise sous vide (74) ;
un boîtier collecteur (60) définissant une chambre intérieure (62) en communication par fluide avec la source de vide (52) par l'orifice de mise sous vide (74) et avec le pansement (20) pour collecter les exudats éliminés du lit de la plaie (10) sous l'influence de la source de vide (52) ;
un filtre hydrophobe (76) en communication par fluide avec la source de vide (52) et la chambre intérieure (62) du boîtier collecteur (60), le filtre (76) étant adapté à empêcher les exudats d'atteindre la source de vide (52) tout en permettant le passage de l'air jusqu'à la source de vide (52) ; système **caractérisé en ce qu'**il comprend en outre :
une grille (80) comprenant des composés pour le clivage des protéines présentes dans les exudats, la grille (80) étant placée en position proximale du filtre (76) pour empêcher l'obturation du filtre (76) par les exudats dans le boîtier collecteur (60).

2. Système suivant la revendication 1, dans lequel les composés sont des protéases.

3. Système suivant la revendication 2, dans lequel les protéases sont une de la papaine, la trypsine, la cathepsine, la thermolysine, la plasmine, la pepsine, la chymotrypsine, la carboxypeptidase Y, Glu-C, Asp-N, Lys-C et leurs associations.

4. Système suivant la revendication 1, dans lequel les composés sont des nucléases.

5. Système suivant la revendication 4, dans lequel les nucléases sont une de l'ADN, de l'ARN et de leurs associations.

6. Système suivant la revendication 4, dans lequel les nucléases sont des exo- et/ou endonucléases.

7. Système suivant l'une quelconque des revendications précédentes, dans lequel la grille (80) est liée mécaniquement au filtre (76).

8. Système suivant l'une quelconque des revendications précédentes, dans lequel la grille (80) est disposée en position adjacente au filtre (76).

9. Système suivant l'une quelconque des revendications précédentes, dans lequel le filtre (76) et la grille (80) sont disposés dans un orifice d'admission de boîtier (72).

10. Système suivant la revendication 9, dans lequel le filtre (76) est disposé de manière substantiellement plane par rapport à l'orifice d'admission de boîtier (72).

11. Système suivant la revendication 10, dans lequel la grille (180) est disposée avec une légère courbure par rapport au filtre (76).

12. Système suivant l'une quelconque des revendications précédentes, dans lequel les composés sont immobilisés sur la grille (80, 180) par un du revêtement de poudre, du revêtement cristallin et du moulage.
